# EUROPÄISCHE PATENTSCHRIFT

(11) **EP 2 364 294 B1**
(45) Veröffentlichungstag und Bekanntmachung des Hinweises auf die Patenterteilung: **03.07.2013**
(21) Anmeldenummer: 09747828.3
(22) Anmeldetag: 04.11.2009
(51) Int. Cl.: C07C 263/10, C07C 265/00

(54) **VERFAHREN ZUR HERSTELLUNG VON ISOCYANATEN**
METHOD FOR PRODUCING ISOCYANATES
PROCÉDÉ DE FABRICATION D'ISOCYANATES

(30) Priorität: 07.11.2008 EP 08168617
(43) Veröffentlichungstag der Anmeldung: 14.09.2011
(73) Patentinhaber: BASF SE, 67056 Ludwigshafen (DE)
(72) Erfinder: MATTKE, Torsten, 67251 Freinsheim (DE); KNÖSCHE, Carsten, 67150 Niederkirchen (DE)
(86) Internationale Anmeldenummer: PCT/EP2009/064578
(87) Internationale Veröffentlichungsnummer: WO 2010/052230

(56) Entgegenhaltungen:
- EP-A1- 1 935 875
- EP-A1- 1 935 876
- WO-A1-2008/055899

## Beschreibung

Die Erfindung geht aus von einem Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt und in einem Reaktor zum Isocyanat umgesetzt werden, wobei ein den Reaktor verlassendes, Isocyanat und Chlorwasserstoff enthaltendes Reaktionsgas in einem Quenchapparat durch Zugabe eines flüssigen Quenchmediums abgekühlt wird, wobei ein Gemisch aus Reaktionsgas und Quenchmedium entsteht.

Die Herstellung von Isocyanaten durch Phosgenierung der korrespondierenden Amine kann prinzipiell durch eine Flüssigphasen- oder eine Gasphasenphosgenierung erfolgen. Die Gasphasenphosgenierung zeichnet sich dadurch aus, dass eine höhere Selektivität möglich und ein geringerer Hold-up an toxischem Phosgen sowie ein verminderter Energiebedarf erforderlich sind.

Bei der Gasphasenphosgenierung werden ein aminhaltiger Eduktstrom und ein phosgenhaltiger Eduktstrom jeweils in gasförmigem Zustand vermischt. Das Amin und das Phosgen setzen sich unter Freisetzung von Chlorwasserstoff (HCl) zu den entsprechenden Isocyanaten um. Der aminhaltige Eduktstrom liegt im Allgemeinen in flüssiger Phase vor und muss vor der Vermischung mit dem phosgenhaltigen Strom verdampft und gegebenenfalls überhitzt werden.

Entsprechende Verfahren zur Herstellung von Isocyanaten in der Gasphase sind zum Beispiel in EP-A 1 319 655 oder EP-A 1 555 258 beschrieben.

Um Folgereaktionen zu vermeiden, ist es notwendig, das Reaktionsgemisch nach Reaktionsende schnell abzukühlen. Hierzu wird zum Beispiel ein Flüssigkeitsquench eingesetzt. Ein solcher Flüssigkeitsquench ist beispielsweise in EP-A 1 403 248 oder in DE-A 10 2006 058 634 beschrieben. Das Quenchmedium, das zur Kühlung zugegeben wird, weist dabei eine Temperatur auf, die im Bereich von 50 bis 200°C liegt. Durch den eingedüsten Flüssigkeitsstrom kühlt das Reaktionsgas schnell auf Temperaturen im Allgemeinen zwischen 100 und 200°C ab. Dabei entsteht ein Zwei-Phasen-Gemisch mit einer isocyanatreichen Flüssigphase und einer isocyanatarmen Gasphase. Beide werden anschließend einer gemeinsamen oder gegebenenfalls separaten Trennstufe, zum Beispiel eine Destillationsstufe zur Trennung von Chlorwasserstoff und Phosgen auf der einen Seite und Isocyanat auf der anderen Seite zugeführt.

Nachteil an den bekannten Vorrichtungen zum Quenchen ist jedoch, dass einige Wandregionen innerhalb des Quenchapparates eine geringe Flüssigkeitsbenetzung aufweisen. An diesen Stellen kann das auskondensierende Reaktionsgemisch lange verweilen und Folgereaktionen unterliegen, die sich negativ auf die Selektivität des Gesamtprozesses auswirken. Zudem können Komponenten, die sublimieren, oder auch durch Folgereaktionen entstandene Feststoffe zu Ablagerungen führen und sich damit negativ auf die Laufzeit der Anlage auswirken. Ein weiteres Problem ist die Entstehung von Aerosolen. Dabei handelt es sich um feinste Tröpfchen oder Partikel, die durch das schnelle Abkühlen der Gasphase entstehen. Diese Aerosole können mit der Gasphase die anschließenden Trennprozesse durchlaufen und vermindern deren Trennwirkung. Daher ist es notwendig, durch entsprechende technische Maßnahmen die Tröpfchen abzutrennen.

Aufgabe der vorliegenden Erfindung ist es somit, ein Verfahren zur Herstellung von Isocyanaten durch Umsetzung der korrespondierenden Amine mit Phosgen bereitzustellen, bei dem die Aerosolbildung und Ablagerungen im Quenchraum vermieden werden.

Gelöst wird die Aufgabe durch ein Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium entsteht. Die zugegebene Menge an Quenchmedium ist so bemessen, dass die sich im Quench einstellende Temperatur des Gemisches aus Reaktionsgas und Quenchmedium oberhalb des Taupunktes des im Quench enthaltenen Gases liegt.

Durch die Bemessung des Quenchmediums derart, dass die Temperatur des Gemischs aus Reaktionsgas und Quenchmedium oberhalb des Taupunktes des im Quench enthaltenen Gases liegt, können sich keine Aerosole bilden. Es kondensieren keine Bestandteile des Gemischs aus Reaktionsgas und Quenchmedium aus bzw. es desublimieren keine Komponenten.

Durch die Vermeidung der Aerosolbildung ist es weiterhin nicht erforderlich, dass das Gas nach dem Quench einen Trennprozess durchlaufen muss, in dem Aerosole abgetrennt werden. Zudem wird die Trennwirkung von Trennprozessen zur Abtrennung des Isocyanats aus dem Gemisch in sich an den Quench anschließenden Trennprozessen nicht vermindert.

Zur Herstellung des Isocyanates werden das Phosgen und das Amin vorzugsweise zunächst einer Mischzone zugeführt, in der die Vermischung von Amin und Phosgen zu einem Reaktionsgemisch erfolgt. Anschließend wird das Reaktionsgemisch dem Reaktor zugeführt, in dem die Umsetzung zum Isocyanat erfolgt. Die Umsetzung von Amin und Phosgen im Reaktor erfolgt dabei vorzugsweise in der Gasphase. Hierzu liegt der Druck im Reaktor vorzugsweise im Bereich zwischen 0,3 bis 3 bar absolut, besonders bevorzugt im Bereich von 0,8 bis 3,0 bar absolut. Die Temperatur liegt dabei vorzugsweise im Bereich von 250 bis 550°C, insbesondere im Bereich von 300 bis 500°C.

Um die Reaktion in der Gasphase durchführen zu können, ist es weiterhin bevorzugt, das Amin und das Phosgen gasförmig zuzugeben. Hierzu weist das Amin vorzugsweise eine Temperatur im Bereich von 200 bis 400°C auf. Der Druck des zugegebenen Amins liegt dabei vorzugsweise im Bereich zwischen 0,05 bis 3 bar absolut. Die Temperatur des zugegebenen Phosgens liegt vorzugsweise im Bereich von 250 bis 450°C. Hierzu wird das Phosgen üblicherweise vor Zugabe auf dem Fachmann bekannte Weise erwärmt.

Zur Erwärmung des Phosgens und des Amins und zur Verdampfung des Amins wird zum Beispiel eine elektrische Beheizung oder eine direkte oder indirekte Aufheizung durch Verbrennung eines Brennstoffs eingesetzt. Als Brennstoffe werden üblicherweise Brenngase, beispielsweise Erdgas, eingesetzt. Durch die Absenkung der Siedetemperatur durch Absenkung des Drucks des Amins ist jedoch auch eine Beheizung, zum Beispiel durch Wasserdampf, möglich. In Abhängigkeit von der Siedetemperatur des Amins wird hierbei der Druck des Wasserdampfes ausgewählt. Ein geeigneter Dampfdruck des Wasserdampfes liegt zum Beispiel im Bereich von 40 bis 100 bar. Daraus ergibt sich eine Temperatur des Wasserdampfes im Bereich von 250 bis 311°C.

Im Allgemeinen ist es erforderlich, das Amin mehrstufig auf die Reaktionstemperatur zu erwärmen. Im Allgemeinen wird das Amin hierzu zunächst vorgewärmt, dann verdampft und anschließend überhitzt. Im Allgemeinen erfordert die Verdampfung die längsten Verweilzeiten und führt so zu Zersetzungen des Amins. Um dies zu minimieren, ist eine Verdampfung bei niedrigeren Temperaturen, wie es sich zum Beispiel durch den niedrigeren Druck ergibt, vorteilhaft. Um nach der Verdampfung das verdampfte Amin auf Reaktionstemperatur zu überhitzen, ist im Allgemeinen eine Beheizung mit Wasserdampf nicht ausreichend. Zur Überhitzung wird daher üblicherweise eine elektrische Beheizung oder eine direkte oder indirekte Aufheizung durch Verbrennung eines Brennstoffs eingesetzt.

Im Unterschied zur Verdampfung des Amins erfolgt die Verdampfung des Phosgens im Allgemeinen bei deutlich niedrigeren Temperaturen. Aus diesem Grund kann zur Verdampfung des Phosgens im Allgemeinen Wasserdampf eingesetzt werden. Jedoch ist auch die notwendige Überhitzung des Phosgens, um dieses auf Reaktionstemperatur zu erwärmen, im Allgemeinen nur durch eine elektrische Beheizung oder eine direkte oder indirekte Aufheizung durch Verbrennung eines Brennstoffes möglich.

Der Reaktor, der zur Phosgenierung des Amins zur Herstellung von Isocyanaten eingesetzt wird, ist dem Fachmann bekannt. Im Allgemeinen werden als Reaktor Rohrreaktoren eingesetzt. Im Reaktor wird das Amin mit dem Phosgen zum korrespondierenden Isocyanat und Chlorwasserstoff umgesetzt. Üblicherweise wird das Phosgen in Überschuss zugegeben, so dass das im Reaktor entstehende Reaktionsgas neben dem gebildeten Isocyanat und dem Chlorwasserstoff auch Phosgen enthält.

Amine, die zur Herstellung von Isocyanaten eingesetzt werden können, sind Monoamine, Diamine, Triamine oder höherwertige Amine. Bevorzugt werden Monoamine oder Diamine eingesetzt. Entsprechend des eingesetzten Amins ergeben sich die korrespondierenden Monoisocyanate, Diisocyanate, Triisocyanate oder höherwertigen Isocyanate. Bevorzugt werden Monoisocyanate oder Diisocyanate mit dem erfindungsgemäßen Verfahren hergestellt.

Diamine und Diisocyanate können aliphatisch, cycloaliphatisch oder aromatisch sein. Bevorzugt sind die Amine aliphatisch oder cycloaliphatisch, besonders bevorzugt aliphatisch.

Cycloaliphatische Isocyanate sind solche, die mindestens ein cycloaliphatisches Ringsystem enthalten.

Aliphatische Isocyanate sind solche, die ausschließlich Isocyanatgruppen aufweisen, die an gerade oder verzweigte Ketten gebunden sind.

Aromatische Isocyanate sind solche, die mindestens eine an mindestens ein aromatisches Ringsystem gebundene Isocyanatgruppe aufweisen.

Im Folgenden wird die Bezeichnung (cyclo)aliphatische Isocyanate für cycloaliphatische und/oder aliphatische Isocyanate verwendet.

Beispiele für aromatische Mono- und Diisocyanate sind bevorzugt solche mit 6 bis 20 C-Atomen, beispielsweise Phenylisocyanat, monomeres 2,4'- und/oder 4,4'-Methylendi (phenylisocyanat) (MDI), 2,4- und/oder 2,6-Toluylendiisocyanat (TDI) und 1,5- oder 1,8-Naphthyldiisocyanat (NDI).

Beispiele für (cyclo)aliphatische Diisocyanate sind aliphatische Diisocyanate wie 1,4-Tetramethylendiisocyanat, 1,6-Hexamethylendiisocyanat (1,6-Diisocyanatohexan), 1,8-Octamethylendüsocyanat, 1,10-Decamethylendiisocyanat, 1,12-Dodecamethylendiisocyanat, 1,14-Tetradecamethylendiisocyanat, 1,5-Diisocyanatopentan, Neopentandiisocyanat, Derivate des Lysindiisocyanats, Tetramethylxylylendiisocyanat (TMXDI), Trimethylhexandiisocyanat oder Tetramethylhexandiisocyanat, sowie 3(beziehungsweise 4)-8(beziehungsweise 9)-Bis(isocyanatomethyl)-tricyclo-[5.2.1.0^{2.6}]-decan-Isomerengemische, sowie cycloaliphatische Diisocyanate wie 1,4-, 1,3- oder 1,2-Diisocyanatocyclohexan, 4,4'- oder 2,4'-Di(isocyanatocyclohexyl)methan, 1-Isocyanato-3,3,5-trimethyl-5-(isocyanatomethyl)cyclohexan (Isoforondiisocyanat), 1,3- oder 1,4-Bis(isocyanatomethyl)cyclohexan, 2,4- oder 2,6-Diisocyanato-1-methylcyclohexan.

Bevorzugte (cyclo)aliphatische Diisocyanate sind 1,6-Diisocyanatohexan, 1-Isocyanato-3,3,5-trimethyl-5-(isocyanato-methyl)cyclohexan, 4,4'-Di(isocyanatocyclohexyl)methan und Toluylendiisocyanat-Isomerengemische. Besonders bevorzugt sind 1,6-Diisocyanatohexan, 1-Isocyanato-3,3,5-trimethyl-5-(isocyanatomethyl)cyclohexan und 4,4'-Di(isocyanatocyclohexyl)me-than.

Amine, die bei dem erfindungsgemäßen Verfahren zur Reaktion zu den korrespondierenden Isocyanaten eingesetzt werden, sind solche, bei denen das Amin, die korrespondierenden Zwischenprodukte und die korrespondierenden Isocyanate bei den gewählten Reaktionsbedingungen gasförmig vorliegen. Bevorzugt sind Amine, die sich während der Dauer der Reaktion unter den Reaktionsbedingungen zu höchstens 2 Mol-%, besonders bevorzugt zu höchstens 1 Mol-% und ganz besonders bevorzugt zu höchstens 0,5 Mol-% zersetzen. Besonders geeignet sind hier Amine, insbesondere Diamine, auf Basis von aliphatischen oder cycloaliphatischen Kohlenwasserstoffen mit 2 bis 18 Kohlenstoffatomen. Beispiele hierfür sind 1,6-Diaminohexan, 1,5-Diaminopentan, 1,3-Bis(aminomethyl)cyclohexan, 1-Amino-3,3,5-trimethyl-5-amino-methylcyclohexan (IPDA) und 4,4-Diaminodicyclohexylmethan. Bevorzugt verwendet wird 1,6-Diaminohexan (HDA).

Ebenfalls können für das erfindungsgemäße Verfahren aromatische Amine verwendet werden, die ohne signifikante Zersetzung in die Gasphase überführt werden können. Beispiele für bevorzugte aromatische Amine sind Toluylendiamin (TDA), als 2,4- oder 2,6-Isomer oder als Gemisch davon, beispielsweise als 80:20 bis 65:35 (Mol/Mol)-Gemisch, Diaminobenzol, 2,6-Xylidin, Naphthyldiamin (NDA) und 2,4'- oder 4,4'-Methylen(diphenyldiamin) (MDA) oder Isomerengemische davon. Bevorzugt sind unter diesen die Diamine, besonders bevorzugt sind 2,4- und/oder 2,6-TDA oder 2,4' - und/oder 4,4'-MDA.

Zur Herstellung von Monoisocyanaten können ebenfalls aliphatische, cycloaliphatische oder aromatische Amine, üblicherweise Monoamine, eingesetzt werden. Als aromatisches Monoamin ist insbesondere Anilin bevorzugt.

Bei der Gasphasenphosgenierung ist es anzustreben, dass die im Reaktionsverlauf auftretenden Verbindungen, also Edukte (Amin und Phosgen), Zwischenprodukte (insbesondere die intermediär entstehenden Mono- und Dicarbamoylchloride), Endprodukte (Diisocyanat), sowie gegebenenfalls zudosierte inerte Verbindungen, unter den Reaktionsbedingungen in der Gasphase verbleiben. Sollten diese oder andere Komponenten sich aus der Gasphase zum Beispiel an der Reaktorwand oder anderen Apparatebauteilen abscheiden, so kann durch diese Abscheidungen der Wärmeübergang oder die Durchströmung der betroffenen Bauteile unerwünscht verändert werden. Dies gilt insbesondere für Auftreten der Aminhydrochloride, die sich aus freien Aminogruppen und Chlorwasserstoff bilden, da die resultierenden Aminhydrochloride leicht ausfallen und nur schwer wieder verdampfbar sind.

Neben dem Einsatz eines Rohrreaktors ist es auch möglich, im Wesentlichen quaderförmige Reaktionsräume, beispielsweise Plattenreaktoren zu verwenden. Auch jeder beliebige andere Querschnitt des Reaktors ist möglich.

Um die Bildung von Nebenprodukten zu vermeiden, ist es bevorzugt, Phosgen im Überschuss zuzuführen. Um nur den für die Reaktion notwendigen Anteil an Aminen zuzuführen, ist es möglich, das Amin mit einem Inertgas zu mischen. Durch den Anteil an Inertgas im Amin lässt sich die Menge des zugeführten Amins bei vorgegebener Geometrie der Zufuhröffnungen für das Amin und das Phosgen einstellen. Inertmedien, die zugegeben werden können, sind solche, die im Reaktionsraum gasförmig vorliegen und nicht mit den im Reaktionsverlauf auftretenden Verbindungen reagieren. Als Inertmedium können zum Beispiel Stickstoff, Edelgase wie Helium oder Argon, Aromaten wie Chlorbenzol, o-Dichlorbenzol, Trichlorbenzol, Toluol, Xylol, Chlornaphtalin, Decahydronaphtalin, Kohlenstoffdioxid oder Kohlenstoffmonoxid eingesetzt werden. Bevorzugt werden jedoch Stickstoff und/oder Chlorbenzol als Inertmedium verwendet.

Alternativ ist es jedoch auch möglich, zum Beispiel um einen zu großen Überschuss an Phosgen zu vermeiden, das Inertmedium dem Phosgen zuzumischen.

Im Allgemeinen wird das Inertmedium in einer Menge zugesetzt, dass das Verhältnis der Gasvolumina von Inertmedium zu Amin beziehungsweise zu Phosgen weniger als 0,0001 bis 30, bevorzugt weniger als 0,01 bis 15 und besonders bevorzugt weniger als 0,1 bis 5 beträgt.

Um die Bildung unerwünschter Nebenprodukte zu reduzieren beziehungsweise zu vermeiden und weiterhin auch eine Zersetzung des gebildeten Isocyanats zu unterbinden, wird das Reaktionsgas unmittelbar nach der Reaktion in einem Quench abgekühlt. Hierzu wird ein vorzugsweise flüssiges Quenchmedium zugegeben. Durch Verdampfung des Quenchmediums nimmt dieses Wärme auf und führt zu einer schnellen Abkühlung des Reaktionsgases.

Um zu vermeiden, dass Reaktionsprodukte oder Reaktionsnebenprodukte oder Quenchmedium auskondensiert oder desublimiert und so Aerosole im Gemisch bildet, ist die Menge an Quenchmedium, die zugegeben wird, so bemessen, dass die sich einstellende Mischtemperatur aus Reaktionsgas und Quenchmedium oberhalb des Taupunktes dieses Gasgemisches liegt. Dafür ist es erforderlich, dass der Partialdruck für alle im Gasgemisch enthaltenen Komponenten jeweils kleiner als deren Dampfdruck ist. Am Taupunkt erreicht der Partialdruck mindestens einer Komponente gerade deren Dampfdruck.

Das Quenchmedium wird im Allgemeinen flüssig zugegeben. Die Temperatur des Quenchmediums liegt dabei vorzugsweise im Bereich von 0 bis 250°C, insbesondere im Bereich von 20 bis 220°C. Durch das Eindüsen des Quenchmediums in das heiße Reaktionsgas wird das Quenchmedium erwärmt und verdampft. Die für das Erwärmen und die Verdampfung des Quenchmediums notwendige Wärme wird dem Reaktionsgas entnommen und das Reaktionsgas wird auf diese Weise abgekühlt. Dadurch, dass die Menge des zugegebenen Quenchmediums so bemessen ist, dass die Temperatur der Mischung aus Reaktionsgas und Quenchmedium oberhalb des Taupunkts dieser Mischung liegt, wird gewährleistet, dass das gesamte Quenchmedium verdampft und nicht Tröpfchen an Quenchmedium im Reaktionsgas verbleiben.

In einer Ausführungsform der Erfindung können sich an den Quench weitere Stufen zur Abkühlung des Reaktionsgases anschließen. In den einzelnen Stufen zur Abkühlung erfolgt dabei jeweils eine weitere Abkühlung des Reaktionsgases bis zum Erreichen der gewünschten Endtemperatur, mit der das Reaktionsgas beispielsweise einer nachfolgenden Aufarbeitung zugeführt wird.

Die weiteren Stufen zur Abkühlung, die sich an den Quench anschließen können, können z.B. weitere Quenche oder Kondensatoren oder beliebige andere Stufen zur Abkühlung, die dem Fachmann bekannt sind, sein. Bevorzugt ist mindestens eine der sich an den Quench anschließenden Stufen zur Abkühlung des Reaktionsgases ein Kondensator. Als Kondensator eignet sich dabei jede beliebige, dem Fachmann bekannte Kondensatorbauart. Üblicherweise wird als Kondensator ein Wärmetauscher eingesetzt, der von einem Kühlmedium durchströmt wird. Als Kühlmittel kann z.B. Wasser eingesetzt werden. In diesem Fall kondensiert das Gas zumindest teilweise an den Wandungen des Kondensators aus. Die so entstehende Flüssigkeit läuft ab und wird gesammelt und dem Kondensator entnommen.

Nach dem Kondensieren des Gemisches aus Reaktionsgas und Quenchmedium wird im Allgemeinen eine Aufbereitung angeschlossen. So ist es z.B. möglich, dass das auskondensierte Gemisch in einem Lösungsmittel gewaschen wird. Als Lösungsmittel können z.B. die gleichen Stoffe eingesetzt werden, die auch als Quenchmedium eingesetzt werden können.

Bei der Wäsche wird das Isocyanat selektiv in die Waschlösung überführt. Anschlie-βend wird bevorzugt durch Rektifikation das enthaltene Gemisch in Isocyanat, Lösungsmittel, Phosgen und Chlorwasserstoff aufgetrennt.

Alternativ zur Abkühlung und Kondensation des Gemisches aus Quenchmedium und Reaktionsgas ist es auch möglich, dass das Gemisch aus Reaktionsgas und Quenchmedium nach Austritt aus dem Quench einer Trennstufe zugeführt wird. Eine entsprechende Trennstufe kann sich alternativ jedoch auch z.B. dem Kondensator anschlie-βen. Bevorzugt schließt sich jedoch die Trennstufe direkt an den Quench an. Als Trennstufen eignen sich z.B. Destillationskolonnen oder Wäscher.

Wenn die Trennstufe ein Wäscher ist, so wird das den Quench verlassende Gemisch aus Reaktionsgas und Quenchmedium vorzugsweise - wie zuvor beschrieben - mit einem Lösungsmittel gewaschen. Hierbei wird das Isocyanat selektiv in die Waschlösung überführt. An die Wäsche schließt sich dann eine Trennung, bevorzugt mittels Rektifikation, an.

Wenn die Trennstufe eine Destillationskolonne ist, so wird das gasförmige Gemisch aus Reaktionsgas und Quenchmedium der Rektifikationskolonne zugeführt. Die Rektifikationskolonne wird so betrieben, dass die Temperatur am Kopf der Rektifikationskolonne niedriger ist als die Siedetemperatur des Gemischs aus Quenchmedium und Reaktionsgas. Auf diese Weise kondensieren in der Destillationskolonne selektiv einzelne Bestandteile des Gemisches aus Quenchmedium und Reaktionsgas aus und können der Kolonne am Sumpf, über Kopf und gegebenenfalls über Seitenabzüge entnommen werden.

Wenn die Trennstufe ein Wäscher ist, so eignet sich insbesondere ein Waschturm, in dem aus dem gasförmigen Gemisch aus Reaktionsgas und Quenchmedium das gebildete Isocyanat durch Kondensation in einem inerten Lösungsmittel abgetrennt wird, während überschüssiges Phosgen, Chlorwasserstoff und gegebenenfalls das Inertmedium den Waschturm gasförmig durchlaufen. Bevorzugt wird dabei die Temperatur des inerten Lösungsmittels oberhalb der Lösungstemperatur des zum Amin gehörigen Carbamoylchlorids im gewählten Waschmedium gehalten. Besonders bevorzugt wird dabei die Temperatur des inerten Lösungsmittels oberhalb der Schmelztemperatur des zum Amin gehörigen Carbamoylchlorids gehalten.

Als Wäscher eignet sich jeder beliebige, dem Fachmann bekannte Wäscher. So können z.B. Rührbehälter oder andere herkömmliche Apparaturen, beispielsweise Kolonnen oder Mixer-Settler-Apparaturen eingesetzt werden.

Das Waschen und die Aufarbeitung des den Quench verlassenden Gemischs aus Reaktionsgas und Quenchmedium erfolgt dabei im Allgemeinen wie beispielsweise in WO-A 2007/028715 beschrieben.

### Beispiel

In einem Reaktor zur Phosgenierung von Aminen zur Herstellung von Isocyanaten werden ca. 1,8 kg/h Toluylendiamin phosgeniert. Das zur Phosgenierung erforderliche Phosgen wird im Überschuss zugeführt. Hierbei entstehen 15,95 kg/h eines Reaktionsgases, das eine Temperatur von 458°C aufweist. Das Reaktionsgas enthält 15,7 Gew.-% Toluylendiisocyanat, 71,2 Gew.-% Phosgen und 13,1 Gew.-% Chlorwasserstoff. Das Reaktionsgas wird in einem Quench mit 7,5 kg/h Monochlorbenzol als Quenchmedium abgekühlt. Das Monochlorbenzol wird flüssig mit einer Temperatur von 60°C über eine Sprühdüse zugeführt. Im Quench herrscht ein Druck von 2 bar absolut. Die sich einstellende Mischtemperatur im Quench liegt bei ca. 195°C. Da der Taupunkt des Reaktionsgases bei 183°C und der des gasförmigen Quenchaustrages bei 178°C liegt, bleibt das den Quench verlassende Gemisch aus Reaktionsgas und Quenchmedium gasförmig, ohne dass sich Aerosole bilden. Dadurch kann der Quench über einen langen Zeitraum störungsfrei betrieben werden. Mit Hilfe eines Schauglases wurde der Quenchaustrag auf Aerosole untersucht. Es konnten keine Aerosole im Quenchaustrag festgestellt werden.

## Patentansprüche

1. Verfahren zur Herstellung von Isocyanaten durch Umsetzung der korrespondierenden Amine mit Phosgen in der Gasphase, gegebenenfalls in Gegenwart eines Inertmediums, bei dem das Amin und das Phosgen zunächst gemischt werden und in einem Reaktor zum Isocyanat umgesetzt werden, wobei ein den Reaktor verlassendes, Isocyanat und Chlorwasserstoff enthaltendes Reaktionsgas in einem Quench durch Zugabe eines flüssigen Quenchmediums abgekühlt wird, wobei ein Gemisch aus Reaktionsgas und Quenchmedium entsteht, **dadurch gekennzeichnet, dass** die zugegebene Menge an Quenchmedium so bemessen ist, dass die sich im Quench einstellende Temperatur des Gemischs aus Reaktionsgas und Quenchmedium oberhalb des Taupunktes des im Quench enthaltenen Gases liegt.

2. Verfahren gemäß Anspruch 1, **dadurch gekennzeichnet, dass** das Amin und dass Phosgen gasförmig zugegeben werden.

3. Verfahren gemäß Anspruch 1 oder 2, **dadurch gekennzeichnet, dass** das Quenchmedium über mindestens eine Düse zugegeben wird.

4. Verfahren gemäß einem der Ansprüche 1 bis 3, **dadurch gekennzeichnet, dass** das Quenchmedium ein gegebenenfalls mit Halogen substituierter Kohlenwasserstoff ist.

5. Verfahren gemäß einem der Ansprüche 1 bis 4, **dadurch gekennzeichnet, dass** das Quenchmedium ausgewählt ist aus der Gruppe bestehend aus Monochlorbenzol, Dichlorbenzol, Trichlorbenzol, Hexan, Benzol, 1,3,5-Trimethylbenzol, Nitrobenzol, Anisol, Chlortoluol, o-Dichlorbenzol, Diethylisophthalat, Tetrahydrofuran, Dimethylformamid, Xylol, Chlomaphthalin, Decahydronaphthalin und Toluol.

6. Verfahren gemäß einem der Ansprüche 1 bis 5, **dadurch gekennzeichnet, dass** sich an den Quench weitere Stufen zur Abkühlung des Reaktionsgases anschließen.

7. Verfahren gemäß Anspruch 6, **dadurch gekennzeichnet, dass** mindestens eine der sich an den Quench anschließenden Stufen zur Abkühlung des Reaktionsgases ein Kondensator ist.

8. Verfahren gemäß einem der Ansprüche 1 bis 5, **dadurch gekennzeichnet, dass** das Gemisch aus Reaktionsgas und Quenchmedium nach Austritt aus dem Quench einer Trennstufe zugeführt wird.

9. Verfahren gemäß Anspruch 8, **dadurch gekennzeichnet, dass** die Trennstufe eine Destillationskolonne oder ein Wäscher ist.

## Claims

1. A process for preparing isocyanates by reacting the corresponding amines with phosgene in the gas phase, optionally in the presence of an inert medium, in which the amine and the phosgene are first mixed and converted to the isocyanate in a reactor, by cooling a reaction gas which comprises isocyanate and hydrogen chloride and leaves the reactor in a quench by adding a liquid quench medium to obtain a mixture of reaction gas and quench medium, wherein the amount of quench medium added is such that the temperature of the mixture of reaction gas and quench medium which is established in the quench is above the dew point of the gas present in the quench.

2. The process according to claim 1, wherein the amine and the phosgene are added in gaseous form.

3. The process according to claim 1 or 2, wherein the quench medium is added via at least one nozzle.

4. The process according to any one of claims 1 to 3, wherein the quench medium is an optionally halogen-substituted hydrocarbon.

5. The process according to any one of claims 1 to 4, wherein the quench medium is selected from the group consisting of monochlorobenzene, dichlorobenzene, trichlorobenzene, hexane, benzene, 1,3,5-trimethylbenzene, nitrobenzene, anisole, chlorotoluene, o-dichlorobenzene, diethyl isophthalate, tetrahydrofuran, dimethylformamide, xylene, chloronaphthalene, decahydronaphthalene and toluene.

6. The process according to any one of claims 1 to 5, wherein the quench is followed by further stages for cooling the reaction gas.

7. The process according to claim 6, wherein at least one of the stages for cooling the reaction gas which follow the quench is a condenser.

8. The process according to any one of claims 1 to 5, wherein the mixture of reaction gas and quench medium is fed to a separation stage after leaving the quench.

9. The process according to claim 8, wherein the separation stage is a distillation column or a scrubber.

## Revendications

1. Procédé pour la préparation d'isocyanates par transformation des amines correspondantes avec du phosgène en phase gazeuse, le cas échéant en présence d'un milieu inerte, dans lequel l'amine et le phosgène sont d'abord mélangés et transformés dans un réacteur en isocyanate, un gaz de réaction, quittant le réacteur, contenant de l'isocyanate et du chlorure d'hydrogène, étant refroidi dans un refroidisseur par addition d'un agent de refroidissement liquide, un mélange de gaz de réaction et de milieu de refroidissement se formant, **caractérisé en ce que** la quantité d'agent de refroidissement ajouté est à dimensionner de manière telle que la température du mélange de gaz de réaction et d'agent de refroidissement, qui se règle dans le refroidisseur, est supérieure au point de rosée du gaz contenu dans le refroidisseur.

2. Procédé selon la revendication 1, **caractérisé en ce que** l'amine et le phosgène sont ajoutés sous forme gazeuse.

3. Procédé selon la revendication 1 ou 2, **caractérisé en ce que** l'agent de refroidissement est ajouté via au moins un gicleur.

4. Procédé selon l'une quelconque des revendications 1 à 3, **caractérisé en ce que** l'agent de refroidissement est un hydrocarbure le cas échéant substitué par halogène.

5. Procédé selon l'une quelconque des revendications 1 à 4, **caractérisé en ce que** l'agent de refroidissement est choisi dans le groupe constitué par le monochlorobenzène, le dichlorobenzène, le trichlorobenzène, l'hexane, le benzène, le 1,3,5-triméthylbenzène, le nitrobenzène, l'anisol, le chlorotoluène, l'o-dichlorobenzène, l'isophtalate de diéthyle, le tétrahydrofuranne, le diméthylformamide, le xylène, le chloronaphtalène, le décahydronaphtalène et le toluène.

6. Procédé selon l'une quelconque des revendications 1 à 5, **caractérisé en ce que** d'autres étapes pour le refroidissement du gaz de réaction sont situées en aval du refroidisseur.

7. Procédé selon la revendication 6, **caractérisé en ce qu'**au moins une des étapes situées en aval du refroidisseur pour le refroidissement du gaz de réaction est un condensateur.

8. Procédé selon l'une quelconque des revendications 1 à 5, **caractérisé en ce que** le mélange du gaz de réaction et d'agent de refroidissement est alimenté à la sortie du refroidisseur dans une étape de séparation.

9. Procédé selon la revendication 8, **caractérisé en ce que** l'étape de séparation est une colonne de distillation ou un laveur.
